(19) Europäisches Patentamt

European Patent Office

Office européen des brevets

(11) **EP 0 658 592 B1**

(12) **FASCICULE DE BREVET EUROPEEN**

(45) Date de publication et mention
de la délivrance du brevet:
**06.02.2002 Bulletin 2002/06**

(51) Int Cl.⁷: **C08K 5/07**, C08K 11/00,
C07C 45/45, C08L 57/08

(21) Numéro de dépôt: **94402697.0**

(22) Date de dépôt: **25.11.1994**

(54) **Composition stabilisante pour polymère chloré comportant des béta-dicétones**

Beton-Diketone enthaltende Stabilisatorzusammensetzung für Chlorpolymer

Stabiliser composition for chlorinated polymer containing beta-diketones

(84) Etats contractants désignés:
**AT BE CH DE DK ES FR GB GR IE IT LI LU NL PT
SE**

(30) Priorité: **15.12.1993 FR 9315064**

(43) Date de publication de la demande:
**21.06.1995 Bulletin 1995/25**

(73) Titulaire: **RHODIA CHIMIE
92512 Boulogne Billancourt Cedex (FR)**

(72) Inventeurs:
• **Allas, Michel
F-79500 Melle (FR)**
• **Mur, Gilles
F-94100 Saint Maur des Fosses (FR)**
• **Chassaing, Serge
F-79500 Melle (FR)**
• **Gay, Michel
F-69100 Villeurbanne (FR)**

(74) Mandataire: **Seugnet, Jean Louis et al
Rhodia Services, Direction de la Propriété
Industrielle, 40, rue de la Haie-Coq
93306 Aubervilliers Cedex (FR)**

(56) Documents cités:
**EP-A- 0 046 161        US-A- 4 102 839**

• **CHEMICAL ABSTRACTS, vol. 118, no. 7, 15
Février 1993, Columbus, Ohio, US; abstract no.
59367h, page 810 ; & J. PRAKT. CHEM. / CHEM.
ZTG., vol.334, no.5, 1992, GERMANY pages 435
- 436 W. MICKLER ET AL. 'By-products of
classical beta-diketone-syntheses'**

## Description

La présente invention concerne une composition stabilisante pour polymère chloré comportant des béta-dicétones ainsi que les objets conformes obtenus à partir desdites compositions .

Les béta-dicétones sont une classe de composés qui bénéficient d'applications commerciales très diverses en particulier dans l'extraction de métaux et dans la stabilisation de polymères chlorés.

Les béta-dicétones sont aujourd'hui les meilleurs stabilisants organiques aisément disponibles pour les polymères chlorés tels que le PVC. C'est pourquoi ces composés sont devenus de plus en plus importants sur le plan commercial. US-A-4 102 839 décrit la stabilisation du PVC, par des $\beta$-dicétones. Les $\beta$-dicétones sont obtenues par condensation d'un ester sur une cétone en présence d'un agent alcalin et d'un solvant. Le mélange réactionnel est acidifié. Le produit est avant son utilisation purifié par recristallisation.

Le coût de fabrication des béta-dicétones peut cependant freiner leur développement. En effet, il est tout à fait économiquement impensable d'utiliser des stabilisants trop coûteux dans l'industrie des polymères chlorés (PVC).

Après avoir effectué de longues et coûteuses recherches, la Demanderesse a trouvé de façon totalement inattendue qu'une composition stabilisante pour polymère chloré (PVC) comportant le produit brut non purifié résultant de la réaction de condensation d'un ester sur une cétone en présence d'un agent alcalin, ce produit brut comportant entre 10 % et 95 %, de préférence entre 20 et 80 % en poids de béta-dicétone et étant sous forme d'une poudre, présente, à poids identique, une action stabilisante au moins égale à celle qu'aurait eu une composition stabilisante comportant de la béta-dicétone purifiée recristallisée, toute chose étant égale par ailleurs.

Cette réaction de condensation d'un ester sur une cétone peut s'écrire :

$$R1COCHR2H + R3\ CO - OR4 + ACat$$

$$R1COCHR2COR3 + R4OH$$

avec :

A Cat est choisi parmi un amidure d'un cation ou un hydrure d'un cation.

R1 et R3, qui peuvent être semblables ou différents, représentent chacun un radical hydrocarboné, ayant avantageusement de 1 à 30 atomes de carbone, de préférence de 1 à 18 atomes de carbone ;

R2 est un hydrogène ou un radical hydrocarboné, en général alkyle, présentant avantageusement au plus 4 atomes de carbone ;

R1 et R2 pouvant être relié de manière que la béta-dicétone forme un cycle ;

R4 représente un radical hydrocarboné.

En ce qui concerne la valeur des radicaux R1, R2 et R3, on peut utiliser un large spectre de radicaux ainsi :
R1 et R3, qui peuvent être identiques ou différents représentant :

- un radical alkyle ou alcényle, linéaire ou ramifié ayant jusqu'à 24 atomes de carbone,

- un radical aralkyle ayant de 7 à 10 atomes de carbone

- un radical aryle ou cycloaliphatique ayant moins de 14 atomes de carbone, les radicaux cycloaliphatiques pouvant éventuellement comporter des liaisons doubles carbone-carbone.

Ces radicaux pouvant être substitués ou non, par exemple par des atomes d'halogène ou, pour des radicaux aryles ou cycloaliphatiques, par des radicaux méthyle ou éthyle. Les radicaux précédemment énumérés peuvent aussi être modifiés par la présence dans la chaîne aliphatique d'un ou plusieurs des groupements de formule :

$$-O-, -CO-O- , -CO-$$

R1 et R3 peuvent également représenter, ensemble, un radical unique divalent ayant de 2 à 5 atomes de carbone et pouvant comporter un hétéroatome d'oxygène ou d'azote ; R2 peut être :

- un atome d'hydrogène (cas préféré)

- un radical alkyle, substitué ou non, ayant de 1 à 4 atomes de carbone et pouvant être interrompu par des groupes -O-, -CO-O, -CO-

R4 représentant un radical alkyle ayant de 1 à 4 atomes de carbone, de préférence méthyle.

La cation Cat est généralement un métal alcalin, de préférence le sodium.

La réaction de condensation décrite ci-dessus est bien connue de l'homme du métier. Elle est plus particulièrement décrite notamment dans les publications suivantes :

**R. HAUSER et Coll.** "The acylation of ketones to form diketones" ORGANIC REACTIONS - Vol. VII Chapter 3 p. 59 - 196 John WILER Ed. NEW-YORK 1954

**WIEDMAN et Coll.** C.R. 238 (1954) p.699

**MORGAN et Coll.** Ber. 58 (1925) p.333

**LIVINGSTONE et Coll.** Am.Soc 46 (1924) p. 881 - 888

**LEVINE Robert et Coll.** Am.Soc. 67 (1945) p.1510 - 1517

qui sont incorporés comme références dans la présente description.

Selon un mode de réalisation préféré, le choix des réactifs de départ est basé sur la disponibilité de ces produits et sur l'activité des produits réactionnels obtenus ; on utilise ainsi un ester comme produit de départ préféré :

- pour l'ester, du stéarate de méthyle notamment de qualité technique pouvant alors contenir comme impureté d'autres esters d'acide gras dont en particulier, le palmitate de méthyle,

- pour l'acétone, l'acétophénone et,

- pour l'agent alcalin, l'amidure de sodium.

On recommande d'utiliser 2 moles d'amidure par mole d'ester ou de cétone engagé et d'utiliser un léger excès (entre 5 et 30 %) molaire de cétone par rapport à l'ester.

Du fait de la présence d'amidure de sodium il est préférable d'effectuer la réaction sous atmosphère inerte, de préférence sous balayage d'azote.

La réaction seffectue à une température située de préférence entre 30 et 60°C.

Si la température s'effectue à la température ambiante (20°C), la cinétique est trop lente.

En outre si la température est trop élevée, par exemple à 60°C etplus, une telle température favorise d'une part, l'autocondensation des cétones en général et de l'acétophenone en particulier et, d'autre part, la formation d'amides.

Les solvants utilisables sont les solvants inertes du genre ether, notamment l'ether isopropylique, les hydrocarbues aliphatiques (par exemple le cyclohexone) ou bien les hydrocarbures aromatiques (toluène).

Bien qu'il soit techniquement possible de conduire la réaction sous une pression plus élevée que la pression atmosphérique, on préfère, pour des motifs économiques, travailler à pression atmosphérique ou bien sous pression réduite de manière à abaisser les températures indiquées ci-dessus et les amener à un domaine compris entre 35 et 55°C. On utilise rarement des pressions inférieures à $10^4$Pa.

En fin de réaction le milieu doit être acidifié. Pour ce faire on coule la solution réactionnelle dans une solution aqueuse d'un acide dont les préférés sont l'acide acétique, l'acide chlorhydrique et l'acide sulfurique. On règle le pH de la couche aqueuse à une valeur comprise de préférence entre 1 et 3.

Pour l'introduction des réactifs, on peut en particulier envisager les 3 modes opératoires suivants :

a) on forme au préalable, l'anion énolite de l'acétophénone en coulant l'acétophénone dans le mélange amidure/ solvant, puis on additionne l'ester,

b) on charge le solvant, l'amidure et la totalite de l'ester, puis on coule lentement l'acétophénone,

c) on coule simultanément l'acétophénone et l'ester dans le mélange amidure/solvant.

On recommande d'utiliser la voie b) puis d'acidifier le mélange réactionnel dans un excès molaire (1,2 à 2 fois molaire) d'acide sulfurique dilué à 5-20 % dans l'eau de façon à ce que le pH soit d'environ 1,5.

Après au moins un lavage à l'eau, on élimine le solvant par tout moyen convenable, par exemple par évaporation et on obtient un produit brut solide à température ambiante comportant généralement entre 40 % et 90 % en poids de béta-dicétone.

Selon l'invention on a découvert que ce produit brut, une fois broyé et réduit à l'état de poudre, est directement utilisable comme additif dans une composition stabilisante pour polymère chloré.

Cette poudre présente une granulométrie généralement inférieure à 500 μm, de préférence inférieure à 200 μm.

Selon une variante particulièrement intéressante de l'invention ce produit brut est broyé suivant une granulométrie silimaire à celle de l'additif stabilisant supplémentaire le plus fin contenu dans la composition stabilisante. Cet additif peut être par exemple un carbonate et/ou un sulfate d'aluminium et/ou de magnésium, par exemple du type hydrotalcite et présenter alors une granulométrie inférieure à 100 μm comprise généralement entre 20 et 100 μm ou bien être un stéarate de calcium et/ou de baryum et présenter alors également une granulométrie très inférieure à 100 μm, comprise généralement entre 50 et 100 μm.

De façon à aboutir à la granulométrie souhaitée, on peut utiliser toutes les techniques connues de l'homme du métier pour obtenir des poudres et notamment :

    a) la précipitation dans un solvant,
    b) le broyage cryogénique,
    c) la pulvérisation/séchage dans un courant froid à contre courant ou à cocourant.

Selon la technique a) on dissout le produit brut solide de la réaction dans un solvant approprié comme par exemple l'éthanol ou le méthanol à température ambiante, on distille le solvant sous une pression réduite de l'ordre de $10^3$Pa puis on injecte de l'azote.

Selon la technique b) on introduit dans un broyeur de l'azote liquide et le produit brut réactionnel sous forme de morceaux de quelques mm à quelques cm obtenus par broyage grossier ou bien par la technique "d'écaillage" de la solution réactionnelle. "L'écaillage" permet d'éliminer le solvant de cette addition réactionnelle par passage de la solution sur un tambour tournant refroidi en permanence. Le produit solidifié à la surface du tambour est récupéré par un racle sous forme précisément "d'écailles".

A la place d'azote liquide, on peut utiliser d'autre gaz liquide inerte tel que le $CO_2$ liquide.

Selon la technique c) on pulvérise le produit brut réactionnel à l'état fondu à travers un contrecourant ou un cocourant d'un gaz inerte vis-à-vis du produit tel que de l'air appauvri en benzène. On récupère des microbilles de produit dont la granulométrie peut être sans difficulté inférieure à 100 μm et allez jusqu'à 10 μm.

Selon une technique bien connue le produit réactionnel brut peut être recristallisé dans un solvant organique approprié, généralement l'éthanol. Le produit recristallisé, séparé par simple filtration des jus mères, se présente sous forme de poudre et est essentiellement formé de béta-dicétones. Pour certaines applications il est nécessaire d'utiliser des béta-dicétones purifiées. L'utilisation des béta-dicétones purifiées à plus de 95 % en poids dans les compositions stabilisantes pour polymère greffé, ne fait pas partie de la présente invention.

Par contre selon l'invention on a trouvé que les jus mères, après élimination du solvant de cristallisation par toute méthode adaptée (par exemple par évaporation où par la technique d'écaillage citée ci-dessus), conduisent à un résidu solide de recristallisation comportant alors généralement au moins 10 % et le plus souvent entre 20 et 40 % de béta-dicétones et que ce résidu de cristallisation, mis également sous forme de poudre, présente, à poids identique, une action stabilisante sur les polymères sensiblement égale à celle qu'auraient eu le même poids d'une composition stabilisante à base de béta-dicétone purifiée recristallisée.

Ces résidus lourds solides de recristallisation sont utilisés de la même façon que le produit brut réactionnel et sont mis en poudre par les mêmes méthodes.

Une stabilisation acceptable des polymères chlorés et plus particulièrement du PVC nécessite le plus souvent l'utilisation conjointe de plusieurs stabilisants qui agissent de manière complémentaire et parfois synergétique.

Ainsi les compositions stabilisantes selon l'invention, outre le produit brut réactionnel ou les résidus lourds de recristallisation sous forme de poudre peuvent contenir une quantité éfficace d'au moins un additif notamment choisi parmi :

    a) un sulfate et/ou un carbonate d'aluminium et/ou de magnésium notamment du type hydrotalcite. De tels produits sont par exemple décrits dans les brevets US-A-4 299 759, US-A-4 427 816, EP-A-453 379 et EP-A-509 864,

    b) un alcool ou un polyol organique conformément à l'enseignement de FR-A-2 356 674 et FR-A-2 356 695

    c) un composé du plomb comme ceux notamment décrits dans ENCYCLOPEDIA of PVC de Leonard I. NASS (1976) pages 299 - 303.

d) un sel d'un métal choisi parmi le calcium, le baryum, le magnesium et le strondium (EP-A-391 311)

e) un composé organique du zinc (EP-A-391 811)

f) un phosphate organique notamment des phosphates de trialkyle ou d'alkyle et de phényle ou de triphényle (EP-A-391 811)

g) un composé organostannique (EP-A-509 864)

h) une huile polyorganosiloxane (EP-A-509 864)

i) des époxydes qui sont généralement des composés complexes, habituellement des polyglycérides époxydée, l'huile de lin époxydée, les huiles de poisson époxydées

j) des adjuvants usuels tels que des antioxydants phénoliques, des agents anti-UV tels que les benzophénons, les benzotriozoles ou les amines stériquement encombrées (habituellement connues sous le terme de Hals.).

De manière générale tout type de PVC convient, quel que soit son mode de préparation : polymérisation en masse, en suspension, en émulsion ou de tout autre type et quelle que soit sa viscosité intrinsèque.

Les homopolymères du chlorure de vinyle peuvent également être modifiés chimiquement, par exemple par chloration. De nombreux copolymères du chlorure de vinyle peuvent également être stabilisés contre les effets de la chaleur, c'est-à-dire le jaunissement et la dégradation. Ce sont en particulier les copolymères obtenus par copolymérisation du chlorure de vinyle avec d'autres monomères présentant une liaison éthylénique polymérisable, comme par exemple l'acétate de vinyle ou le chlorure de vinylidène ; les acides maléiques ou fumarique ou leurs esters ; les oléfines telles que l'éthylène, le propylène, l'hexène ; les esters acryliques ou méthacryliques ; le styrène ; les éthers vinyliques tels que le vinyldodécyléther.

Habituellement ces copolymères contiennent au moins 50 % en poids de motifs chlorure de vinyle et de préférence au moins 80 % en poids de motifs chlorure de vinyle.

Les compositions selon l'invention peuvent également contenir des mélanges à base de polymère chloré contenant des quantités minoritaires d'autres polymères, comme les polyoléfines halogénées ou les copolymères acrylonitrile-butadiène-styrène.

Le PVC seul ou en mélange avec d'autres polymères est le polymère chloré le plus largement utilisé dans les compositions de l'invention.

Les compositions de l'invention peuvent être des formulations rigides, c'est-à-dire sans plastifiant, ou semi-rigides, c'est à dire avec des teneurs en plastifiant réduites, telles que pour les applications dans le bâtiment la fabrication de profilés divers ou cablerie électrique, ou pour les compositions ne contenant que des additifs alimentaires, pour la fabrication de bouteilles.

Ces formulations contiennent le plus souvent un renforçateur de chocs, tel qu'un copolymère méthacrylate/butadiène/styrène par exemple.

Elles peuvent également être des formulations plastifiées telles que pour la fabrication de films à usage agricole.

Les plastifiants utilisés dont des composés connus tels que par exemple des phtalates d'alkyle. Le plus souvent utilisé est le phtalate de di(éthyl-2hexyle) (habituellement appelé phtalate de dioctyle).

Lorsque les compositions contiennent un plastifiant, la teneur de celui-ci est généralement de 5 % à 120 % en poids par rapport au poids de polymère chloré.

La présence de carbonate basique d'aluminium et le magnésium dans les compositions de l'invention apportent un certain nombre d'avantages.

Ainsi dans les compositions contenant des composés du plomb, le carbonate basique d'aluminium et de magnésium de formule (i) permet de réduire la quantité du composé du plomb et éventuellement du cadmium lorsqu'il est présent, sans diminuer la stabilité thermique du polymère, ce qui est un élément positif, car on remplace partiellement des composés qui présentent une certaine toxicité par un composé potentiellement alimentaire.

Dans les compositions contenant des composés organo-stanniques la présence de carbonate basique d'aluminium et de magnésium de formule (i) permet de diminuer le cas échéant la quantité de composé époxydé, qui est généralement un composé liquide. Le remplacement partiel d'un composé liquide permet d'améliorer la rigidité et le degré VICAT (indice de résistance aux chocs) des objets conformes préparés à partir des compositions rigides.

De manière habituelle, l'incorporation des différents stabilisants ou adjuvants est faible sur le polymère chloré à l'état de poudre.

On peut bien entendu préparer un mélange de 2 ou plusieurs des composés constitutifs des compositions selon l'invention avant leur incorporation dans le polymère chloré.

Toutes les méthodes usuelles d'incorporation des différents stabilisants ou adjuvants dans le polymère peuvent être utilisées. Par exemple l'homogénéisation de la composition polymérique peut être réalisée sur malaxeur ou mélangeur à rouleaux, à une température telle que la composition devienne fluide, normalement entre 150°C et 200°C pour le PVC et pendant une durée suffisante, de l'ordre de quelques minutes à quelques dizaines de minutes.

Les compositions de polymère chloré, et plus particulièrement de PVC, peuvent être mises en oeuvre selon toutes les techniques utilisées habituellement comme par exemple l'extrusion, l'injection, l'extrusion-soufflage, le calandrage ou le moulage par rotation.

Les exemples qui suivent illustrent l'invention.

Dans ce qui suit, ou ce qui précède sauf indications expresses contraires, les parties et pourcentages sont en poids.

**EXEMPLE 1 : Préparation du produit brut réactionnel P1.**

Dans un réacteur de 2000 cm3 muni d'un réfrigérant, d'une bonne agitation et avec la possibilité d'être relié soit au vide soit à une source d'azote on introduit :

    Toluène        260 ml

puis sous couverture d'azote :

    NaNH2        78 g

La température du milieu est ensuite portée à 40°C, puis maintenue durant toute la réaction et la finition à cette température.

L'ensemble de l'appareillage est mis sous une pression de $7\ 10^4$Pa.

On coule 310 g de stéarate de méhyle technique (contenant 10 % de palmitate de méthyle).

En 3 heures, on coule 120 g d'acétophénone.

La coulée de l'acétophénone étant terminée, on laisse sous agitation pendant 45 mn le milieu réactionnel (température de 40°C et sous pression de $7\ 10^4$Pa).

La solution toluénique est ensuite coulée à chaud dans une solution d'acide sulfurique diluée à 10 % de façon que le pH de la couche aqueuse après décantation soit de 1,5.

Après deux lavages, la solution toluénique est ensuite évaporée par passage de la solution sur un tambour tournant refroidi en permanence pour conduire à un produit brut P1 sous forme "d'écailles" soit 420 g d'un produit solide à 20°C titrant 78 % en béta-dicétones (rendement 82 %).(analyse chonolographique CPG)

**EXEMPLE 2 : Préparation des résidus lourds L1.**

On répète exactement le mode opératoire de l'exemple 1. Le produit brut réactionnel obtenu exempt de toluène est dissous dans 1500 ml d'éthanol.

On filtre et on essore sur verre fritté n°2.

Puis on effectue successivement 2 lavages par 500 ml d'éthanol suivi d'un essorage.

On obtient 304 g d'un produit pur PP1 titrant 97 % en poids de béta-dicétones.

On obtient un produit appelé produit lourd L1 présentant un point de fusion d'environ 50°C et titrant 16 % en poids de béta-dicétones (analyse chronolographique CPG).

**EXEMPLE 3 : Préparation d'une poudre P1.**

Dans un réacteur agité sphérique muni de moyen de refroidissement et d'une agitation tripale en fond de cuve, on introduit à 20°C 3 parties d'éthanol à 7 % d'eau et on coule en 1 heure 1,0 parties de P1 préparé selon le mode opératoire de l'exemple 1 et chauffé à 70°C. On maintient la température du contenu du réacteur entre 20 et 25°C. Le réacteur est mis sous une pression réduite de $2\ 10^3$Pa. et l'éthanol est distillé en prenant garde que la température ne dépasse pas 25°C dans la masse. On injecte ensuite de l'azote jusqu'à disparition des traces d'eau. On obtient une poudre jaune clair P1 dont la granulométrie est d'environ 120 μm.

**EXEMPLE 4 : Préparation d'une poudre L1.**

On répète exactement le mode opératoire de l'exemple 3, sauf qu'on remplace 1 partie de produit P1 de départ par 1 partie de lourd L1 obtenu selon le mode opératoire de l'exemple 2. On obtient ainsi une poudre marron dont la granulométrie est d'environ 130 μm.

### EXEMPLE 5 : Préparation d'une poudre P1.

Dans un broyeur à martezau équipé d'une grille de 1 mm, on introduit au moyen d'un transporteur à vis 1 partie/heure de produit P1 de l'exemple 1 et 0,3 partie/heure d'azote liquide. Le produit broyé est récupéré par un transport pneumatique vers un système de séparation à filtre et l'on obtient une poudre blanche de granulométrie de 50 μm.

On obtient une poudre analogue en remplaçant P1 par L1 et l'azote liquide par du $CO_2$ liquide.

### EXEMPLE 6 : Préparation d'une poudre P1.

En tête d'une tour de "prilling" fonctionnant à contre - courant on alimente, au travers d'une couronne de pulvérisateurs 1 partie/heure de produit P1 à 70°C obtenu selon l'exemple 1. On fait tourner à contre courant environ 10 parties/heure d'air à 8 % (en volume) en oxygène maintenu à une température comprise entre 15 et 20°C. On récupère au pied de la tour 1 partie/heure de P1 en microbulles sphériques dont la granulométrie est de 40 μm. L'air appauvri en oxygène est recyclé à la base de la tour après filtration et refroidissement à 15/20°C.

On obtient une poudre dont la granulométrie est de 100 μm. Un résultat analogue est obtenu en remplaçant P1 par L1.

### Exemple 7 :

On part d'une composition de PVC semi-rigide en poudre ayant la composition suivante :

- PVC poudre préparée par polymérisation en suspension et commercialisé sous la dénomination SOLVIC 271 GB        100 parties
- DOP (dioctylphtalate)        25 parties
- Ethyl-2 hexanoate de calcium        0,4 parties
- Octoate de zinc        0,15 parties
- phosphite OS 150® (diphenylisododécyl-phosphite)        1,0 partie
- Cire de polyéthylène polaire PE 191® commercialisée par HOECHST        1,0 partie
- irganox 1076® antioxydant commercialisé par CIBA-CEIGY        0,1 partie
- huile de soja époxydée        3 parties

A partir de cette composition qui sert de témoin on réalise différents échantillons en ajoutant pour 100 parties de résine PVC, par homogénéisation pendant 10 mn à l'aide du mélangeur de laboratoire CNTA B17, 0,1, 0,2 et 0,3 parties respectivement de poudre L1 obtenue selon l'exemple 5 ainsi que de poudre PP1 cristallisée obtenue à l'exemple 2.

A partir des poudres ainsi obtenues on prépare des feuilles de 1 mm d'épaisseur par passage pendant 10 mn à 180°C sur un malaxeur à 2 cylindres.

Dans les feuilles ainsi obtenues on découpe des éprouvettes de 27,5 cm de longueur et de 2 cm de largeur. Celles-ci sont ensuite disposées sur un plateau mobile introduit dans un four à 190°C et sorties à vitesse constante (0,45 cm/mn) pendant une heure. Ceci permet d'obtenir la dégradation thermique de l'éprouvette en fonction du temps de séjour (Test METRASTAT).

On quantifie la dégradation thermique en mesurant à l'aide d'un chromamètre MINOLTA CR 200® sur le standart (Y, x, y) l'évolution de l'indice de jaune en fonction du temps. Ceci permet d'obtenir trois paramètres significatifs de comparaison à savoir :

- la coloration initiale (CI) qui est l'indice de jaune à 2,5 mm d'exposition (Incertitude $\Delta Y = 1,5$) ;
- la stabilité de coloration (SC) (évaluée en minute), qui correspond au temps pour lequel l'indice de jaune varie de 2 points (Incertitude $\Delta T = 2$ mn).
- la stabilité thermique long terme (STLT) (évaluée en minutes) ou temps de cramage qui est le temps ou apparait la dégradation (noircissement) de l'éprouvette (Incertitude $\Delta T = 4$ mn).

Les résultats obtenus sont rassemblés dans le tableau 1 ci-après.

De l'examen du tableau 1, il apparait que l'utilisation de L1 (lourds de recristallisation) donne des effets supérieurs à PP1. Ce phénomère est d'autant plus marqué pour la stabilité de coloration.

En outre comme P1, L1 améliore la stabilité thermique long terme.

TABLEAU 1

| Additif en partie pour cent partie de résine PVC | C.I (%) | S.C. (min) | STLT (min) |
|---|---|---|---|
| Témoin | 13,6 | 7,8 | 37 |
| 0,1 PP1[+] | 9,4 | 20 | 33 |
| 0,2 PP1[+] | 8,8 | 18,2 | 33 |
| 0,3 PP1[+] | 9,8 | 21 | 37 |
| 0,1 L1 | 10,2 | 20,6 | 36 |
| 0,2 L1 | 10,5 | 22 | 38 |
| 0,3 L1 | 14,7 | 24,8 | 44 |

[+] en dehors de l'invention

**Exemple 8 :**

On part d'une composition en PVC (formulation rigide), ayant la composition suivante :

- PVC poudre préparé par polymérisation en suspension et commercialisé sous la dénomination S110 P (ATOCHEM) :   100 parties
- Stéarate de Calcium, stabilisant commercialisé par ATOCHEM sous la référence STAVINOR PSME   0,7   "
- Stéarate de Zinc, stabilisant commercialisé par ATOCHEM sous la référence ZN 70.   0,8   "
- Didécylphenylphosphite, stabilisant commercialisé par CIBA- GEIGY sous la référence IRGASTAB CH 300   0,8   "
- Alcool polyvinylique, stabilisant commercialisé par ERKOL sous la référence Rhodiastab PVAL   0,2   "
- Hydrotalcyte (hydrogenocarbonate d'Aluminium et de Magnésium), stabilisant commercialisé sous la référence ALCAMIZER 4 par MITSUI   0,2   "
- Carbonate de Calcium broyé, charge commercialisée sous sous la référence OMYALITE 95T par OMYA   9,5   "
- Pigment d'oxyde de Titane, commercialisé par KRONOS sous la référence CL 2220   5   "
- Renforçant choc (polymère acrylique), commercialisé par RHOM et HAAS sous le nom PARALOID KM 355   6,5   "
- Lubrifiants commercialisés par HENKEL sous les noms :

  - LOXIOL G20 (ester d'un diacide aromatique et d'un alcool gras aliphatique)   0,6   "
  - LOXIOL G30 (ester cireux)   0,3   "
  - LOXIOL G21 (Acide-12/ Hydroxystéarique)   0,2   "

A partir de cette composition, on réalise 2 échantillons en ajoutant :

- 0,25 parties du produit P1 de l'exemple 5
- 0,25 parties du produit PP1 de l'exemple 2

Chaque échantillon est homogénéisé pendant 10 minutes à 4500 t/min, à l'aide d'un mélangeur de laboratoire CNTA B17.

A partir de ces poudres, on procède à une stabilité thermique dynamique au plastographe BRABENDER ®. Cet appareillage est constitué :

- d'un système moteur électrique accouplé à un variateur continu ou discontinu de vitesse (0 à 200 t/min).

- d'un bain thermostaté à régulation de température proportionnelle électronique par de l'huile silicone.

- d'un malaxeur, muni d'une cuve à double paroi, permettant un chauffage par circulation de l'huile silicone et de 2 rotors fixés par un système de verrouillage à baïonnette.

- d'un chronomètre.

Chaque essai est introduit dans ce malaxeur à 150°C, à raison de 52 g, à l'aide d'une trémie et d'un piston poussé par un poids de 5 kg. Puis, toutes les 5 minutes, on effectue un prélèvement afin d'obtenir une pastille, jusqu'au noircissement ou cramage du mélange PVC.

Sur chaque pastille sont mesurés par coloration, à l'aide d'un chromamètre MINOLTA CR 200 ®, l'indice de blanc (IB) selon le standard (L,a,b) CIE de la Norme ASTM E313 et l'indice de jaunissement (IJ) qui est mesuré selon le standard (Y,x, y) de la Norme ASTM D1925.

On obtient ainsi la dégradation thermique de chaque formule en fonction du temps.

Les résultats obtenus sont rassemblés dans le tableau 2 ci-après :

TABLEAU 2

| Temps | Produit P1 (exemple 5) | Produit PP1 +(exemple 2) |
|---|---|---|
| 5 min | IJ = 7,3<br>IB = 63,5 | IJ = 6,7<br>IB = 65,3 |
| 10 min | IJ = 7,4<br>IB = 63,7 | IJ = 8,3<br>IB = 62,2 |
| 15 min | IJ = 8,3<br>IB = 61,3 | IJ = 9,2<br>IB = 59,5 |
| 20 min | IJ = 8,1<br>IB = 61,3 | IJ = 9,1<br>IB = 60,0 |
| 25 min | IJ = 8,4<br>IB = 61,1 | CRAMAGE<br>" |
| 30 min | CRAMAGE | |

+ eu deliors de l'invention

L'examen du tableau montre que le produit brut P1 broyé a une stabilité thermique meilleure que le produit recristallisé PP1; son indice de jaune évolue moins vite dans le temps et la blancheur se maintient à des valeurs supérieures à celles obtenues par le produit PP1.

**<u>EXEMPLE 9</u>** :

On part d'une composition de PVC semi-rigide en poudre ayant la composition suivante :

- PVC poudre préparée par polymérisation en suspension et commercialisé sous la dénomination SOLVIC 271 GB      100 parties

- DOP (dioctalphtalate)      25 parties

- Ethyl-2 hexanote de calcium      0,4 parties

- Acetate de zinc      0,15 parties

- phosphate OS 150® (diphenylisodécylph-phosphite)      1,0 partie

- Cire de polyéthylène polaire PE 191® commercialisée par HOECHST      1,0 partie

- Irganox 1076® antioxydant commercialisé par CIBA-CEIGY      0,1 partie

- huile de soja époxydée      3 parties

A partir de cette composition qui sert de témoin on réalise différents échantillons en ajoutant pour 100 parties de résine PVC, par homogénéisation pendant 10 mn à l'aide du mélangeur de laboratoire CNTA B17, 0,1, 0,2 et 0,3 parties respectivement de poudre P1 et L1 obtenues selon l'exemple 5 ainsi que de poudre PP1 cristallisée obtenue à l'exemple 2.

A partir des poudres ainsi obtenues on prépare des feuilles de 1 mm d'épaisseur par passage pendant 10 mn à 180°C sur un malaxeur à 2 cylindres.

Dans les feuilles ainsi obtenues on découpe des éprouvettes de 27,5 cm de longueur et de 2 cm de largeur. Celles-ci sont ensuite disposées sur un plateau mobile introduit dans un four à 190°C et sorties à vitesse constante (0,45 cm/mn) pendant une heure. Ceci permet d'obtenir la dégradation thermique de l'éprouvette en fonction du temps de séjour (Test METRASTAT).

On quantifie la dégradation thermique en mesurant à l'aide d'un chronomètre MINOLTA CR 200® sur le standart (Y, x, y) l'évolution de l'indice de jaune en fonction du temps. Ceci permet d'obtenir trois paramètres significatifs de comparaison à savoir :

- la coloration initiale (CI) qui est l'indice de jaune à 2,5 mm d'exposition (Incertitude $\Delta Y = 1,5$);

- la stabilité de coloration (SC) (évaluée en minute), qui correspond au temps pour lequel l'indice de jaune varie de 2 points (Incertitude $\Delta T = 2$ mn).

- la stabilité thermique évaluée en minutes) long terme (STLT) ou temps de cramage qui est le temps ou apparait la dégradation (noicissement) de l'éprouvette (Incertitude $\Delta T = 4$ mn).

Les résultats obtenus sont rassemblés dans le tableau 1 ci-après.

De l'examen du tableau 1, il apparait que le pouvoir stabilisant de PP1 (produit recristalisé) est, pour un même poids, analogue à celui de P1 (produit de réaction brut non recristallisé), notamment en ce qui concerne la stabilité de coloration, la couleur initiale gardant pratiquement la même évolution.

De plus, on améliore, à poids identique, la stabilité thermique long terme en utilisant P1 à la place de PP1.

On remarque en outre que l'utilisation de L1 (lourds de recristallisation) donne des effets analogues à PP1. Ce phénomère est d'autant plus marqué pour la stabilité de coloration.

En outre comme P1, L1 améliore la stabilité thermique long terme.

TABLEAU 1

| Additif en partie pour cent partie de résine PVC | C.I (%) | S.C. (min) | STLT (min) |
|---|---|---|---|
| Témoin | 13,6 | 7,8 | 37 |
| 0,1 PP1[+] | 9,4 | 20 | 33 |
| 0,2 PP1[+] | 8,8 | 18,2 | 33 |
| 0,3 PP1[+] | 9,8 | 21 | 37 |
| 0,1 P1 | 10,0 | 12,9 | 39 |
| 0,2 P1 | 9,0 | 14,4 | 37,5 |
| 0,3 P1 | 8,9 | 11,5 | 38,5 |
| 0,1 L1 | 10,2 | 20,6 | 36 |
| 0,2 L1 | 10,5 | 22 | 38 |
| 0,3 L1 | 14,7 | 24,8 | 44 |

[+] en dehors de l'invention

**Exemple 10** :

On part d'une composition en PVC (formulation rigide), ayant la composition suivante :

- PVC poudre préparé par polymérisation en suspension et commercialisé sous la dénomination S110 P (ATOCHEM) :       100 parties
- Stéarate de Calcium, stabilisant commercialisé par ATOCHEM sous la référence STAVINOR PSME       0,7       "
- Stéarate de Zinc, stabilisant commercialisé par ATOCHEM sous la référence ZN 70.       0,8       "
- Didécylphenylphosphite, stabilisant commercialisé par CIBA- GEIGY sous la référence IRGASTAB CH 300       0,5       "
- Alcool polyvinylique, stabilisant commercialisé par RP CHIMIE sous la référence Rhodiastab PVAL       0,1       "
- Hydrotalcyte (hydrogenocarbonate d'Aluminium et de Magnésium), stabilisant commercialisé sous la référence ALCAMIZER 4 par MITSUI       0,4       "

- Carbonate de Calcium broyé, charge commercialisée sous sous la référence OMYALITE 95T par OMYA       9,5       "
- Pigment d'oxyde de Titane, commercialisé par KRONOS sous la référence CL 2220        5       "
- Renforçant choc (polymère acrylique), commercialisé par RHOM et HAAS sous le nom PARALOID KM 355       6,5       "
- Lubrifiants commercialisés par HENKEL sous les noms :

    - LOXIOL G60 (ester d'un diacide aromatique et d'un alcool gras aliphatique)        0,4       "
    - LOXIOL G21 (Acide-12/ Hydroxystéarique)        0,2       "

- d'un processing aid (agent de mise en oeuvre) commercialisé par ROHM et HAAS PARALOID KRON        1       "

A partir de cette composition, on réalise 2 échantillons en ajoutant :

- 0,25 parties du produit P1 de l'exemple 5
- 0,25 parties du produit P2 (P2 = stéaroylbenzoylméthane en dehors de la présente invention).

Chaque échantillon est homogénéisé dans un mélangeur rapide type Papenmeier à la vitesse de 1 800 tr/mn jusqu'à une température de 115°C.

A partir de ces poudres, on procède à une transformation par extrusion afin d'obtenir des plaques.

Les caractéristiques de l'extrudeuse monovis sont :

- Fabricant : ANDOUART,
- Vis conique : taux de compression = 2.8
  rapport longueur/diamètre = 20
  diamètre D = 40 mm

Les conditions d'extrusion sont :

- Vitesse de rotation de la vis = 23 tr/mn
- Profil de température :

| Zone 1 | 2 | 3 | Filières |
|--------|------|------|---------|
| 175°C | 180°C | 185°C | 190°C |

Les plaques ainsi extrudées sont soumises à un rayonnement UV dans deux types de conditions :

1) CONDITIONS UVCON

- Appareillage UVCON de ATLAS
- Spectre d'éclairement :
  UVA avec un maximum à a = 340 nm et filtre < 290 nm
- Température de corps noir = 55°C
- Atmosphère saturée d'humidité

2) CONDITIONS XENOTEST
    XENOTEST 1200 CPS

- Eclairement énergétique        -        80 w/m2
- T°C chambre        -        30 °C
- Cycle = 29 min sec        →        65% humidité relative
  = 1 min pluie        →        99% humidité relative
- 3 lampes xénon
- Type de filtre : 3 filtres suprax 1/3
- Corps noir

On obtient ainsi la tenue à la lumière UV de chaque formule en fonction du temps.

Les résultats obtenus sont rassemblés dans le tableau 3 ci-après :

## TABLEAU 3

| | CONDITIONS UV.CON | | | CONDITIONS XENOTEST | |
|---|---|---|---|---|---|
| | P1 | P2 | | P1 | P2 |
| $\Delta$b1 | 10,71 | 17,10 | $\Delta$b2 | 4,31 | 5,73 |

$\Delta$b1 : différence d'indice de jaune entre le temps d'exposition de 600 h et le temps initial.

$\Delta$b2 : différence d'indice de jaune entre le temps d'exposition de 1 000 h et le temps initial.

Du tableau 3, il apparaît que P1, conforme à l'invention, a un indice de jaune qui évolue moins vite dans le temps après exposition à la lumière UV que P2, en dehors de l'invention.

**Revendications**

1. Utilisation comme additif dans une composition stabilisante pour polymère chloré (PVC) d'un produit brut pouvant être obtenu en mettant en oeuvre les étapes suivantes :

   ☐ on effectue une condensation d'un ester sur une cétone en présence d'un agent alcalin, et d'un solvant,
   ☐ on acidifie le mélange réactionnel,
   ☐ on effectue au moins un lavage à l'eau du mélange ainsi obtenu,
   ☐ on évapore le solvant,

   ce produit brut comportant entre 10 et 95 %, de préférence entre 20 et 80 % en poids de béta-dicétone et étant sous forme d'une poudre.

2. Utilisation comme additif dans une composition stabilisante pour polymère chloré (PVC) d'un résidu solide se présentant sous forme de poudre et pouvant être obtenu en mettant en oeuvre les étapes suivantes :

   ☐ on effectue une condensation d'un ester sur une cétone en présence d'un agent alcalin, et d'un solvant,
   ☐ on acidifie le mélange réactionnel,
   ☐ on effectue au moins un lavage à l'eau du mélange ainsi obtenu,
   ☐ on évapore le solvant, pour obtenir un produit brut comportant entre 10 et 95 %, de préférence entre 20 et 80 % en poids de béta-dicétone,
   ☐ on effectue une recristallisation dudit produit brut dans un solvant organique approprié,
   ☐ on sépare le produit recristallisé des jus mères par filtration,
   ☐ on évapore le solvant des jus mères pour obtenir le résidu solide.

3. Utilisation selon la revendication 2, **caractérisée en ce que** le résidu solide de recristallisation comporte au moins 10 % et de préférence entre 20 et 40 % de béta-dicétones.

4. Utilisation selon l'une quelconque des revendications précédentes, **caractérisée en ce que** la réaction de condensation s'effectue suivant la réaction :

$$R^1COCHR^2H + R^3CO\text{-}OR^4 + ACat \rightarrow R^1COCHR^2OR^3 + R^4OH$$

avec :

- A Cat est choisi parmi un amidure d'un cation ou un hydrure d'un cation ;
- $R^1$ et $R^3$, qui peuvent être semblables ou différents, représentent chacun un radical hydrocarboné, ayant avantageusement de 1 à 30 atomes de carbone, de préférence de 1 à 18 atomes de carbone ;
- $R^2$ est un hydrogène ou un radical hydrocarboné, en général alkyle, présentant avantageusement au plus 4 atomes de carbone ;
- $R^1$ et $R^2$ pouvant être relié de manière que la béta-dicétone forme un cycle ;
- $R^4$ représente un radical hydrocarboné.

5.  Utilisation selon la revendication 4 **caractérisée en ce que** :

    $R^1$ et $R^3$, qui peuvent être identiques ou différents représentent :

    - un radical alkyle ou alcényle, linéaire ou ramifié ayant jusqu'à 24 atomes de carbone,
    - un radical aralkyle ayant de 7 à 10 atomes de carbone
    - un radical aryle ou cycloaliphatique ayant moins de 14 atomes de carbone,

    $R^4$ représente un radical alkyle ayant de 1 à 4 atomes de carbone, de préférence méthyle.

6.  Utilisation selon l'une quelconque des revendications précédentes, **caractérisée en ce que** l'ester est le stéarate de méthyle, l'acétone est l'acétophénone et l'agent alcalin est l'amidure de sodium.

7.  Utilisation selon l'une quelconque des revendications 4 à 6, **caractérisée en ce que** la réaction de condensation est effectuée à une température comprise entre 30 et 60°C (à pression atmosphérique).

8.  Utilisation selon l'une quelconque des revendications précédentes **caractérisée en ce que** ladite poudre présente une granulométrie inférieure à 500 µm, de préférence inférieure à 200 µm.

9.  Utilisation selon l'une quelconque des revendications précédentes **caractérisée en ce que** la composition stabilisante comprend au moins un additif stabilisant supplémentaire.

10. Utilisation selon la revendication précédente **caractérisée en ce que** le produit brut se présente sous la forme d'une poudre dont la granulométrie est similaire à celle de l'additif stabilisant supplémentaire.

11. Utilisation selon l'une des revendications 9 ou 10, **caractérisée en ce que** ledit additif supplémentaire est choisi parmi un carbonate et/ou un sulfate d'aluminium et/ou de magnésium, une hydrotalcite, du stéarate de calcium et/ou de baryum et **en ce que** les granulométries de l'additif supplémentaire et du produit brut sont similaires et inférieures à 100 µm.

12. Utilisation selon la revendication 9, **caractérisée en ce que** le résidu solide de recristallisation se présente sous la forme d'une poudre dont la granulométrie est similaire à celle de l'additif stabilisant supplémentaire.

13. Utilisation selon l'une quelconque des revendications précédentes, **caractérisée en ce que** la poudre peut être obtenue à partir du produit brut solide à 20°C, ou du résidu solide de recristallisation, et par la mise en oeuvre d'au moins l'une des techniques suivantes :

    - la précipitation dans un solvant,
    - le broyage cryogénique et,
    - la pulvérisation/séchage dans un courant froid à cocourant ou à contrecourant.

14. Composition stabilisante pour polymère chloré (PVC) comprenant :

    - un produit brut pouvant être obtenu en mettant en oeuvre les étapes suivantes :

      ☐  on effectue une condensation d'un ester sur une cétone en présence d'un agent alcalin, et d'un solvant,
      ☐  on acidifie le mélange réactionnel,
      ☐  on effectue au moins un lavage à l'eau du mélange ainsi obtenu,
      ☐  on évapore le solvant,

ce produit brut comportant entre 10 et 95 %, de préférence entre 20 et 80 % en poids de béta-dicétone et étant sous forme d'une poudre.

- au moins un additif stabilisant supplémentaire.

**15.** Composition stabilisante pour polymère chloré (PVC) comprenant :

- un résidu solide se présentant sous forme de poudre et pouvant être obtenu en mettant en oeuvre les étapes suivantes :

  ☐ on effectue une condensation d'un ester sur une cétone en présence d'un agent alcalin, et d'un solvant,
  ☐ on acidifie le mélange réactionnel,
  ☐ on effectue au moins un lavage à l'eau du mélange ainsi obtenu,
  ☐ on évapore le solvant, pour obtenir un produit brut comportant entre 10 et 95 %, de préférence entre 20 et 80 % en poids de béta-dicétone,
  ☐ on effectue une recristallisation dudit produit brut dans un solvant organique approprié,
  ☐ on sépare le produit recristallisé des jus mères par filtration,
  ☐ on élimine le solvant des jus mères pour obtenir le résidu solide,

  le résidu solide de recristallisation comportant au moins 10 % et de préférence entre 20 et 40 % de béta-dicétones,

- au moins un additif stabilisant supplémentaire, choisi parmi

      a) un sulfate et/ou un carbonate d'aluminium et/ou de magnésium,
      b) un alcool ou un polyol organique,
      c) un composé du plomb,
      d) un sel d'un métal choisi parmi le calcium, le baryum, le magnesium et le strondium,
      e) un composé organique du zinc
      f) un phosphate organique,
      g) un composé organostannique,
      h) une huile polyorganosiloxane,
      i) des époxydes,
      j) des antioxydants phénoliques, des agents anti-UV.

**16.** Composition selon l'une quelconque des revendications 14 ou 15, **caractérisée en ce que** la réaction de condensation s'effectue suivant la réaction :

$$R^1COCHR^2H + R^3CO\text{-}OR^4 + ACat \rightarrow R^1COCHR^2COR^3 + R^4OH$$

avec :

- A Cat est choisi parmi un amidure d'un cation ou un hydrure d'un cation ;
- $R^1$ et $R^3$, qui peuvent être semblables ou différents, représentent chacun un radical hydrocarboné, ayant avantageusement de 1 à 30 atomes de carbone, de préférence de 1 à 18 atomes de carbone ;
- $R^2$ est un hydrogène ou un radical hydrocarboné, en général alkyle, présentant avantageusement au plus 4 atomes de carbone ;
- $R^1$ et $R^2$ pouvant être relié de manière que la béta-dicétone forme un cycle ;
- $R^4$ représente un radical hydrocarboné.

**17.** Composition selon la revendication 16 **caractérisée en ce que** :

$R^1$ et $R^3$, qui peuvent être identiques ou différents représentent :

- un radical alkyle ou alcényle, linéaire ou ramifié ayant jusqu'à 24 atomes de carbone,
- un radical aralkyle ayant de 7 à 10 atomes de carbone
- un radical aryle ou cycloaliphatique ayant moins de 14 atomes de carbone,

$R^4$ représente un radical alkyle ayant de 1 à 4 atomes de carbone, de préférence méthyle.

18. Composition selon l'une quelconque des revendications 14 à 17, **caractérisée en ce que** l'ester est le stéarate de méthyle, l'acétone est l'acétophénone et l'agent alcalin est l'amidure de sodium.

19. Composition selon l'une quelconque des revendications 14 à 18, **caractérisée en ce que** la réaction de condensation est effectuée à une température comprise entre 30 et 60°C (à pression atmosphérique).

20. Composition selon l'une quelconque des revendications 14 à 19, **caractérisée en ce que** le produit brut ou le résidu solide de recristallisation présente une granulométrie, inférieure à 500 µm, de préférence inférieure à 200 µm.

21. Composition selon l'une quelconque des revendications 14 à 20, **caractérisée en ce que** ladite poudre présente une granulométrie similaire à la granulométrie de l'additif stabilisant supplémentaire contenu dans la composition stabilisante.

22. Composition selon la revendication 20, **caractérisé en ce que** l'additif supplémentaire est choisi parmi :

    a) un sulfate et/ou un carbonate d'aluminium et/ou de magnésium,
    b) un alcool ou un polyol organique,
    c) un composé du plomb,
    d) un sel d'un métal choisi parmi le calcium, le baryum, le magnesium et le strondium,
    e) un composé organique du zinc
    f) un phosphate organique,
    g) un composé organostannique,
    h) une huile polyorganosiloxane,
    i) des époxydes,
    j) des antioxydants phénoliques, des agents anti-UV.

23. Composition selon la revendication 14, **caractérisée en ce que** l'additif supplémentaire est choisi parmi un carbonate et/ou un sulfate d'aluminium et/ou de magnésium, une hydrotalcite, du stéarate de calcium et/ou de baryum et **en ce que** les granulométries de l'additif supplémentaire et du produit brut sont similaires et inférieures à 100 µm.

24. Objets obtenus à partir de polymère chloré (PVC) et d'une composition stabilisante comprenant l'additif dont l'utilisation fait l'objet des revendications 1 à 13, ou la composition selon l'une des revendications 14 à 23.

**Patentansprüche**

1. Verwendung eines Rohproduktes als Additiv in einer Stabilisatorzusammensetzung für ein chloriertes Polymer (PVC), wobei das Rohprodukt erhalten werden kann unter Durchführung der folgenden Verfahrensschritte:

• Man führt eine Kondensation eines Esters mit einem Keton in Gegenwart eines alkalischen Reagenzes und eines Lösemittels durch,
• man säuert das Reaktionsmilieu an,
• man führt mindestens eine Wäsche der so erhaltenen Mischung mit Wasser durch,
• man verdampft das Lösemittel,

wobei dieses Rohprodukt 10 bis 95 Gew.-%, vorzugsweise 20 bis 80 Gew.-%, beta-Diketon enthält und in Form eines Pulvers vorliegt.

2. Verwendung eines festen Rückstandes in Form eines Pulvers als Additiv in einer Stabilisatorzusammensetzung für ein chloriertes Polymer (PVC),
wobei der Rückstand erhalten werden kann unter Durchführung der folgenden Verfahrensschritte:

• Man führt eine Kondensation eines Esters mit einem Keton in Gegenwart eines alkalischen Reagenzes und eines Lösemittels durch,
• man säuert das Reaktionsmilieu an,

- man führt mindestens eine Wäsche der so erhaltenen Mischung mit Wasser durch,
- man verdampft das Lösemittel, um ein Rohprodukt zu erhalten, das 10 bis 95 Gew.-%, vorzugsweise 20 bis 80 Gew.-%, beta-Diketon enthält,
- man führt eine Umkristallisierung des Rohproduktes in einem geeigneten organischen Lösemittel durch,
- man trennt das umkristallisierte Produkt durch Filtration aus den Mutterlaugen ab,
- man verdampft das Lösemittel der Mutterlaugen, um den festen Rückstand zu erhalten.

3. Verwendung nach Anspruch 2, **dadurch gekennzeichnet, daß** der feste Rückstand aus der Umkristallisierung mindestens 10 %, vorzugsweise 20 bis 40 %, beta-Diketone enthält.

4. Verwendung nach einem der vorhergehenden Ansprüche, **dadurch gekennzeichnet, daß** die Kondensationsreaktion gemäß der folgenden Reaktion durchgeführt wird:

$$R^1COCHR^2H + R^3CO\text{-}OR^4 + ACat \rightarrow R^1COCHR^2COR^3 + R^4OH$$

wobei:

- ACat ausgewählt ist aus einem Amid eines Kations oder einem Hydrid eines Kations;
- $R^1$ und $R^3$, gleich oder verschieden, jeweils einen Kohlenwasserstoffrest, vorzugsweise mit 1 bis 30 Kohlenstoffatomen, bevorzugt mit 1 bis 18 Kohlenstoffatomen, darstellen;
- $R^2$ Wasserstoff oder einen Kohlenwasserstoffrest, im allgemeinen Alkyl, mit vorzugsweise höchstens 4 Kohlenstoffatomen darstellt;
- $R^1$ und $R^2$ verbunden sein können, so daß das beta-Diketon einen Ring bildet;
- $R^4$ einen Kohlenwasserstoffrest darstellt.

5. Verwendung nach Anspruch 4, **dadurch gekennzeichnet, daß**:

$R^1$ und $R^3$, identisch oder verschieden, darstellen:

- einen linearen oder verzweigten Alkyl- oder Alkenylrest mit bis zu 24 Kohlenstoffatomen,
- einen Aralkylrest mit 7 bis 10 Kohlenstoffatomen,
- einen Arylrest oder einen cycloaliphatischen Rest mit weniger als 14 Kohlenstoffatomen,

$R^4$ einen Alkylrest mit 1 bis 4 Kohlenstoffatomen, vorzugsweise Methyl, darstellt.

6. Verwendung nach einem der vorhergehenden Ansprüche, **dadurch gekennzeichnet, daß** der Ester Methylstearat , das Aceton Acetophenon und das alkalische Reagenz Natriumamid ist.

7. Verwendung nach einem der Ansprüche 4 bis 6, **dadurch gekennzeichnet, daß** die Kondensationsreaktion bei einer Temperatur zwischen 30 und 60 °C (Atmosphärendruck) durchgeführt wird.

8. Verwendung nach einem der vorhergehenden Ansprüche, **dadurch gekennzeichnet, daß** das Pulver eine Granulometrie unterhalb von 500 μm, vorzugsweise unterhalb von 200 μm, aufweist.

9. Verwendung nach einem der vorhergehenden Ansprüche, **dadurch gekennzeichnet, daß** die Stabilisatorzusammensetzung mindestens ein zusätzliches stabilisierendes Additiv enthält.

10. Verwendung nach dem vorhergehenden Anspruch, **dadurch gekennzeichnet, daß** das Rohprodukt in Form eines Pulvers vorliegt, dessen Granulometrie ähnlich der des zusätzlichen stabilisierenden Additivs ist.

11. Verwendung nach einem der Ansprüche 9 oder 10, **dadurch gekennzeichnet, daß** das zusätzliche Additiv ausgewählt ist aus einem Magnesium- und/oder Aluminiumsulfat und/oder -carbonat, einem Hydrotalcid und einem Barium- und/oder Calciumstearat und daß die Granulometrie des zusätzlichen Additivs und des Rohprodukts gleich sind und unterhalb von 100 μm liegen.

12. Verwendung nach Anspruch 9, **dadurch gekennzeichnet, daß** der feste Rückstand aus der Umkristallisierung in Form eines Pulvers vorliegt, dessen Granulometrie gleich der des zusätzlichen stabilisierenden Additivs ist.

13. Verwendung nach einem der vorhergehenden Ansprüche, **dadurch gekennzeichnet, daß** das Pulver ausgehend von einem bei 20 °C festen Rohprodukt oder einem festen Rückstand aus der Umkristallisierung unter Durchführung mindestens einer der folgenden Methoden erhalten sein kann:

- Ausfällen in einem Lösemittel,
- kryogene Zerkleinerung und
- Pulverisierung /Trocknung in einem kalten Strom mit Costrom oder Gegenstrom.

14. Stabilisatorzusammensetzung für ein chloriertes Polymer (PVC), enthaltend:

- ein Rohprodukt, das unter Durchführung der folgenden Verfahrensschritte erhalten werden kann:

  • Man führt eine Kondensation eines Esters mit einem Keton in Gegenwart eines alkalischen Reagenzes und eines Lösemittels durch,
  • man säuert die Reaktionsmischung an,
  • man führt mindestens eine Wäsche der so erhaltenen Mischung mit Wasser durch,
  • man verdampft das Lösemittel,

  wobei das Rohprodukt 10 bis 95 Gew.-%, vorzugsweise 20 bis 80 Gew.-%, beta-Diketon enthält und in Form eines Pulvers vorliegt;

- mindestens ein zusätzliches stabilisierendes Additiv.

15. Stabilisatorzusammensetzung für ein chloriertes Polymer (PVC), enthaltend:

- einen festen Rückstand in Form eines Pulvers, der unter Durchführung der folgenden Verfahrensschritte erhalten werden kann:

  • Man führt eine Kondensation eines Esters mit einem Keton in Gegenwart eines alkalischen Reagenzes und eines Lösemittels durch,
  • man säuert die Reaktionsmischung an,
  • man führt mindestens eine Wäsche der so erhaltenen Mischung mit Wasser durch,
  • man verdampft das Lösemittel, um ein Rohprodukt mit 10 bis 95 Gew.-%, vorzugsweise 20 bis 80 Gew.-%, beta-Diketon zu erhalten,
  • man führt eine Umkristallisierung des Rohproduktes in einem geeigneten organischen Lösemittel durch,
  • man trennt das umkristallisierte Produkt durch Filtration aus den Mutterlaugen ab,
  • man entfernt das Lösemittel aus den Mutterlaugen, um den festen Rückstand zu erhalten,

  wobei der feste Rückstand aus der Umkristallisierung mindestens 10 %, vorzugsweise 20 bis 40 %, beta-Diketon enthält,

- mindestens ein zusätzliches stabilisierendes Additiv, das ausgewählt ist aus

  a) einem Aluminium- und/oder Magnesiumsulfat und/oder -carbonat,
  b) einem organischen Alkohol oder Polyol,
  c) einer Bleiverbindung,
  d) einem Metallsalz, wobei das Metall ausgewählt ist aus Calcium, Barium, Magnesium und Strontium,
  e) einer organischen Zinkverbindung,
  f) einem organischen Phosphat,
  g) einer Organozinnverbindung,
  h) einem Polyorganosiloxanöl,
  i) Epoxiden,
  j) phenolischen Antioxidantien und UV-Schutzreagenzien.

16. Zusammensetzung nach einem der Ansprüche 14 oder 15, **dadurch gekennzeichnet, daß** die Kondensationsreaktion gemäß der folgenden Reaktion durchgeführt wird:

$$R^1COCHR^2H + R^3CO\text{-}OR^4 + ACat \rightarrow R^1COCHR^2COR^3 + R^4OH$$

wobei:

- ACat ausgewählt ist aus einem Amid eines Kations oder einem Hydrid eines Kations;
- $R^1$ und $R^3$, gleich oder verschieden, jeweils einen Kohlenwasserstoffrest, vorzugsweise mit 1 bis 30 Kohlenstoffatomen, bevorzugt mit 1 bis 18 Kohlenstoffatomen, darstellen;
- $R^2$ Wasserstoff oder einen Kohlenwasserstoffrest, im allgemeinen Alkyl, mit vorzugsweise höchstens 4 Kohlenstoffatomen darstellt;
- $R^1$ und $R^2$ verbunden sein können, so daß das beta-Diketon einen Ring bildet;
- $R^4$ einen Kohlenwasserstoffrest darstellt.

17. Zusammensetzung nach Anspruch 16, **dadurch gekennzeichnet, daß**:

$R^1$ und $R^3$, identisch oder verschieden, darstellen:

- einen linearen oder verzweigten Alkyl- oder Alkenylrest mit bis zu 24 Kohlenstoffatomen,
- einen Aralkylrest mit 7 bis 10 Kohlenstoffatomen,
- einen Arylrest oder einen cycloaliphatischen Rest mit weniger als 14 Kohlenstoffatomen,

$R^4$ einen Alkylrest mit 1 bis 4 Kohlenstoffatomen, vorzugsweise Methyl, darstellt.

18. Zusammensetzung nach einem der Ansprüche 14 bis 17, **dadurch gekennzeichnet, daß** der Ester Methylstearat, das Aceton Acetophenon und das alkalische Reagenz Natriumamid ist.

19. Zusammensetzung nach einem der Ansprüche 14 bis 18, **dadurch gekennzeichnet, daß** die Kondensationsreaktion bei einer Temperatur zwischen 30 und 60 °C (Atmosphärendruck) durchgeführt wird.

20. Zusammensetzung nach einem der Ansprüche 14 bis 19, **dadurch gekennzeichnet, daß** das Rohprodukt oder der feste Rückstand aus der Umkristallisierung eine Granulometrie von weniger als 500 µm, vorzugsweise weniger als 200 µm, aufweist.

21. Zusammensetzung nach einem der Ansprüche 14 bis 20, **dadurch gekennzeichnet, daß** das Pulver die gleiche Granulometrie wie die Granulometrie des zusätzlichen stabilisierenden Additivs aufweist, das in der Stabilisatorzusammensetzung enthalten ist.

22. Zusammensetzung nach Anspruch 20, **dadurch gekennzeichnet, daß** das zusätzliche Additiv ausgewählt ist aus:

a) einem Aluminium- und/oder Magnesiumsulfat und/oder -carbonat,
b) einem organischen Alkohol oder Polyol,
c) einer Bleiverbindung,
d) einem Metallsalz, wobei das Metall ausgewählt ist aus Calcium, Barium, Magnesium und Strontium,
e) einer organischen Zinkverbindung,
f) einem organischen Phosphat,
g) einer Organozinnverbindung,
h) einem Polyorganosiloxanöl,
i) Epoxiden,
j) phenolischen Antioxidantien und UV-Schutzreagenzien.

23. Zusammensetzung nach Anspruch 14, **dadurch gekennzeichnet, daß** das zusätzliche Additiv ausgewählt ist aus einem Magnesium- und/oder Aluminiumsulfat und/oder -carbonat, einem Hydrotalcid, einem Calcium- und/oder Bariumstearat und daß die Granulometrie des zusätzlichen Additivs und des Rohproduktes gleich sind und unterhalb von 100 µm liegen.

24. Gegenstände, erhalten ausgehend von einem chlorierten Polymer (PVC) und einer Stabilisatorzusammensetzung, die ein Additiv enthält, dessen Verwendung Gegenstand der Ansprüche 1 bis 13 ist, oder die Zusammensetzung nach einem der Ansprüche 14 bis 23.

**Claims**

1.  Use as additive in a stabilizing composition for a chlorinated polymer (PVC) of a crude product which can be obtained by employing the following stages:

    ☐  an ester is condensed with a ketone in the presence of an alkaline agent and of a solvent,
    ☐  the reaction mixture is acidified,
    ☐  the mixture thus obtained is washed at least once with water,
    ☐  the solvent is evaporated,

    this crude product comprising between 10 and 95%, preferably between 20 and 80%, by weight of β-diketone and being in the form of a powder.

2.  Use as additive in a stabilizing composition for a chlorinated polymer (PVC) of a solid residue which is provided in the form of a powder and which can be obtained by employing the following stages:

    ☐  an ester is condensed with a ketone in the presence of an alkaline agent and of a solvent,
    ☐  the reaction mixture is acidified,
    ☐  the mixture thus obtained is washed at least once with water,
    ☐  the solvent is evaporated, to obtain a crude product comprising between 10 and 95%, preferably between 20 and 80%, by weight of β-diketone,
    ☐  the said crude product is recrystallized from an appropriate organic solvent,
    ☐  the recrystallized product is separated from the mother liquors by filtration,
    ☐  the solvent is evaporated from the mother liquors to obtain the solid residue.

3.  Use according to claim 2, **characterized in that** the solid recrystallization residue comprises at least 10% and preferably between 20 and 40% of β-diketones.

4.  Use according to any one of the preceding claims, **characterized in that** the condensation reaction is carried out according to the reaction:

$$R^1COCHR^2H + R^3CO\text{-}OR^4 + ACat \rightarrow R^1COCHR^2COR^3 + R^4OH$$

    in which:

    -  ACat is chosen from an amide of a cation or a hydride of a cation;
    -  $R^1$ and $R^3$, which can be alike or different, each represent a hydrocarbonaceous radical advantageously having from 1 to 30 carbon atoms, preferably from 1 to 18 carbon atoms;
    -  $R^2$ is a hydrogen or a hydrocarbonaceous radical, generally an alkyl radical, advantageously exhibiting at most 4 carbon atoms;
    -  it being possible for $R^1$ and $R^2$ to be connected so that the β-diketone forms a ring;
    -  $R^4$ represents a hydrocarbonaceous radical.

5.  Use according to claim 4, **characterized in that**:

    $R^1$ and $R^3$, which can be identical or different, represent:

    -  a linear or branched alkyl or alkenyl radical having up to 24 carbon atoms,
    -  an aralkyl radical having from 7 to 10 carbon atoms,
    -  an aryl or cycloaliphatic radical having less than 14 carbon atoms,

    $R^4$ represents an alkyl radical having from 1 to 4 carbon atoms, preferably a methyl radical.

6.  Use according to any one of the preceding claims, **characterized in that** the ester is methyl stearate, the ketone is acetophenone and the alkaline agent is sodium amide.

7.  Use according to any one of claims 4 to 6, **characterized in that** the condensation reaction is carried out at a temperature of between 30 and 60°C (at atmospheric pressure).

8. Use according to any one of the preceding claims, **characterized in that** the said powder exhibits a particle size of less than 500 μm, preferably of less than 200 μm.

9. Use according to any one of the preceding claims, **characterized in that** the stabilizing composition comprises at least one additional stabilizing additive.

10. Use according to the preceding claim, **characterized in that** the crude product is provided in the form of a powder, the particle size of which is similar to that of the additional stabilizing additive.

11. Use according to either of claims 9 and 10, **characterized in that** the said additional additive is chosen from an aluminium and/or magnesium carbonate and/or sulphate, a hydrotalcite, or calcium and/or barium stearate, and **in that** the particle sizes of the additional additive and of the crude product are similar and less than 100 μm.

12. Use according to claim 9, **characterized in that** the solid recrystallization residue is provided in the form of a powder, the particle size of which is similar to that of the additional stabilizing additive.

13. Use according to any one of the preceding claims, **characterized in that** the powder can be obtained from the crude product which is solid at 20°C or from the solid recrystallization residue and by the implementation of at least one of the following techniques:

   - precipitation from a solvent,
   - cryogenic milling and,
   - spraying/drying in a cold stream, cocurrentwise or countercurrentwise.

14. Stabilizing composition for a chlorinated polymer (PVC) comprising:

   - a crude product which can be obtained by employing the following stages:

      ☐ an ester is condensed with a ketone in the presence of an alkaline agent and of a solvent,
      ☐ the reaction mixture is acidified,
      ☐ the mixture thus obtained is washed at least once with water,
      ☐ the solvent is evaporated,

   this crude product comprising between 10 and 95%, preferably between 20 and 80%, by weight of β-diketone and being in the form of a powder,

   - at least one additional stabilizing additive,

15. Stabilizing composition for a chlorinated polymer (PVC) comprising:

   - a solid residue which is provided in the form of a powder and which can be obtained by employing the following stages:

      ☐ an ester is condensed with a ketone in the presence of an alkaline agent and of a solvent,
      ☐ the reaction mixture is acidified,
      ☐ the mixture thus obtained is washed at least once with water,
      ☐ the solvent is evaporated, to obtain a crude product comprising between 10 and 95%, preferably between 20 and 80%, by weight of β-diketone,
      ☐ the said crude product is recrystallized from an appropriate organic solvent,
      ☐ the recrystallized product is separated from the mother liquors by filtration,
      ☐ the solvent is removed from the mother liquors to obtain the solid residue,

   - the solid recrystallization residue comprising at least 10% and preferably between 20 and 40% of β-diketones,
   - at least one additional stabilizing additive chosen from

      a) an aluminium and/or magnesium sulphate and/or carbonate,

b) an organic alcohol or polyol,
c) a lead compound,
d) a salt of a metal chosen from calcium, barium, magnesium and strontium,
e) an organic zinc compound,
f) an organic phosphate,
g) an organotin compound,
h) a polyorganosiloxane oil,
i) epoxides,
j) phenolic antioxidants, UV inhibitors.

16. Composition according to either one of claims 14 and 15, **characterized in that** the condensation reaction is carried out according to the reaction:

$$R^1COCHR^2H + R^3CO\text{-}OR^4 + ACat \rightarrow R^1COCHR^2COR^3 + R^4OH$$

in which:

- ACat is chosen from an amide of a cation or a hydride of a cation;
- $R^1$ and $R^3$, which can be alike or different, each represent a hydrocarbonaceous radical advantageously having from 1 to 30 carbon atoms, preferably from 1 to 18 carbon atoms;
- $R^2$ is a hydrogen or a hydrocarbonaceous radical, generally an alkyl radical, advantageously exhibiting at most 4 carbon atoms;
- it being possible for $R^1$ and $R^2$ to be connected so that the β-diketone forms a ring;
- $R^4$ represents a hydrocarbonaceous radical.

17. Composition according to claim 16, **characterized in that**:

$R^1$ and $R^3$, which can be identical or different, represent:

- a linear or branched alkyl or alkenyl radical having up to 24 carbon atoms,
- an aralkyl radical having from 7 to 10 carbon atoms,
- an aryl or cycloaliphatic radical having less than 14 carbon atoms,

$R^4$ represents an alkyl radical having from 1 to 4 carbon atoms, preferably a methyl radical.

18. Composition according to any one of claims 14 to 17, **characterized in that** the ester is methyl stearate, the ketone is acetophenone and the alkaline agent is sodium amide.

19. Composition according to any one of claims 14 to 18, **characterised in that** the condensation reaction is carried out at a temperature of between 30 and 60°C (at atmospheric pressure).

20. Composition according to any one of claims 14 to 19, **characterized in that** the crude product or the solid recrystallization residue exhibits a particle size of less than 500 µm, preferably of less than 200 µm.

21. Composition according to any one of claims 14 to 20, **characterized in that** the said powder exhibits a particle size similar to the particle size of the additional stabilizing additive present in the stabilizing composition.

22. Composition according to claim 20, **characterized in that** the additional additive is chosen from:

a) an aluminium and/or magnesium sulphate and/or carbonate,
b) an organic alcohol or polyol,
c) a lead compound,
d) a salt of a metal chosen from calcium, barium, magnesium and strontium,
e) an organic zinc compound,
f) an organic phosphate,
g) an organotin compound,
h) a polyorganosiloxane oil,

i) epoxides,
j) phenolic antioxidants, UV inhibitors.

23. Composition according to claim 14, **characterized in that** the additional additive is chosen from an aluminium and/or magnesium carbonate and/or sulphate, a hydrotalcite, or calcium and/or barium stearate, and **in that** the particle sizes of the additional additive and of the crude product are similar and less than 100 µm.

24. Objects obtained from a chlorinated polymer (PVC) and from a stabilizing composition comprising the additive, the use of which forms the subject-matter of claims 1 to 13, or the composition according to one of claims 14 to 23.